# EUROPEAN PATENT APPLICATION

(11) **EP 1 955 722 A1**
(43) Date of publication of application: **13.08.2008**
(21) Application number: 07290160.6
(22) Date of filing: 08.02.2007
(51) Int. Cl.: A61L 27/30, A61L 27/34, A61L 27/42, A61L 27/44, B05D 1/06

(54) **Method for providing a composite film onto a substrate**

(71) Applicant: INSTITUT NATIONAL DE LA SANTE ET DE LA RECHERCHE MEDICALE (INSERM), 75013 Paris (FR)
(72) Inventor: Ball, Vincent, 67500 Marienthal (FR); Voegel, Jean-Claude, 67210 Valff (FR); Schaaf, Pierre, 67120 Molsheim (FR); Ladhari, Nadia, 67100 Strasbourg (FR); Hemmerle, Joseph, 67720 Weyersheim (FR)
(74) Representative: Colombet, Alain André

(57) **Abstract**

The invention relates to a method for providing a composite film onto a substrate comprising the steps consisting of:
i) coating onto a substrate disposed vertically a solution comprising a polycationic compound by spray coating, thus forming a layer A;
ii) coating onto the layer A an aqueous solution of a mineral oxide precursor by spray coating, thus forming a layer B; and
iii) repeating steps i) and ii) until the formed composite film has the desired thickness.

It further relates to a substrate coated with a composite film of alternate layers A and layers B, wherein layer A comprises a polycationic compound and layer B comprises a mineral oxide.

## Description

The present invention relates to a method for providing an organic-inorganic composite film onto a substrate, in particular biomaterials, and to such substrates.

The fabrication of well controlled composite coatings on solid surfaces is a promising new tool to control both the mechanical, optical, wetting and permeability properties of the interface between the solid and its surroundings.

Some methods for producing such composite films are known.

In particular, WO 04/047880 proposes to produce a composite coating of polyelectrolyte and particles of calcium phosphate by immersion.

According to US 3,754,945, a refractory laminate may be prepared on the surface of a support by dipping the support into a bath of colloidal silica or alkaline ionic silicate to form a coating on the substrate, contacting the coated surface with a polycationic organic setting agent and repeating this sequence until the coating has the desired thickness.

Both these methods use immersion of the substrate into a solution as a deposition technique. This technique is however quite costly because it implies the use of large volumes of solution. In order to avoid contamination by residues of the preceding solution, it is further necessary to dry the substrate before proceeding with the next step, which is energy and time consuming, in particular for coating formed by a large number of layers. Finally, the coating obtained generally has an inhomogeneous thickness, and may be opaque in the visible spectrum.

Other deposition techniques such as spray coating are known for producing polyelectrolyte multilayers, for instance built up from hyaluronic acid and poly(L-lysine), (C. Porcel, et al. Langmuir 22(9), 4376-4383, 2006). However, it has proven difficult to use this technique for deposition of mineral oxide precursors. One major reason for this is that even very small particles, like colloidal particles, may lead to the obstruction of the spraying nozzle.

It is therefore one object of the present invention to propose a method for providing a composite film onto a substrate which is simple, fast and not substrate specific, so that it can be used to coat a variety of different substrates.

It is another object of the present invention to propose a method which does not imply the use of organic solvents.

It is yet another object of the present invention to propose a method for providing a composite film onto a substrate wherein the composite film adheres well to the substrate, has a well defined composition, a controlled, homogeneous thickness and a controlled surface roughness.

The present invention is based on the observation that multilayer composite films can be obtained using a layer -by -layer deposition spray coating if the mineral oxide precursor solution is chosen to be stable to allow for its application through a spraying nozzle, but to decompose upon contact with active groups of the underlying polycationic compound to yield oxide particles.

This method allows a rapid and low cost preparation of composite films on a variety of substrates.

The films obtained have a homogeneous thickness which can be finely tuned according to the intended application. Further, the films show a particular behaviour with respect to their permeability towards multivalent ions.

According to a first object, the present invention thus aims at a method for providing a composite film onto a substrate comprising the steps consisting of:
i) coating onto a substrate a solution comprising a polycationic compound by spray coating, thus forming a layer A;
ii) coating onto the layer A an aqueous solution of mineral oxide precursor by spray coating, thus forming a layer B; and
iii) repeating steps i) and ii) until the formed composite film has the desired thickness.

The proposed method is also called hereafter "reactive layer by layer spray coating".

It can be used for a wide variety of different substrates the surface properties of which are to be modified. This includes in particular biomaterials. For instance, it can be useful to coat implants or prosthesis. It can also be used as an antibacterial coating, in particular when including titanium dioxyde (Y. Kikuchi et al. J. Photochem. Photobiol. A 106, 51-56, 1997).

A major feature of the proposed method is the fact that the mineral oxide precursor is decomposed upon contact with the polycationic compound present in the preceding, underlying layer. The contact with the polycationic compound induces a reaction, such as a polycondensation, of the mineral oxide precursor, which yields the mineral oxide.

Indeed, while spray coating of the mineral precursor alone does not lead to a noticeable raise of the film thickness, alternate spray coating of mineral precursor and polycationic compound does lead to the formation of a film whose thickness rises linearly with the number of deposition steps.

Without wishing to be bound by this theory, it is believed that this is due to the reaction between the reactive groups of the polycationic compound such as amine groups and the mineral oxide precursor. This reaction leads to the formation of mineral oxide, in particular in form of particles.

A number of deposition steps are necessary to form a homogeneous film. Generally, this film will be considered as homogeneous upon percolation of the mineral oxide particles. The number of deposition steps necessary to obtain a homogeneous film may depend on a number of process parameters, in particular on the concentration on the solution used. Generally however, at least 10 layer deposition steps (counting either step (i) or step (ii)) are necessary to obtain a homogeneous film.

In fact, the polycationic compounds do not only play the role of a catalyst by enhancing the rate of mineral oxide decomposition but they may also be an active component of the obtained composite material.

Indeed, infrared spectra of composite films obtained according to the invention show that the Si-O stretching band is significantly displaced from 1085 cm⁻¹ in the absence of polycation to 1039 cm⁻¹ for the polycation containing material.

Preferred polycationic compound are hydrosoluble, so as to allow to work in aqueous solutions and to avoid organic solvents.

Particularly preferred polycationic compounds are chosen from polyamines, polyimines and, polyaminoacids, and copolymers comprising amine, imine or aminoacid groups. Amine groups may be aliphatic or aromatic primary, secondary, tertiary or quaternary.

The cationic charge density of these compounds depends essentially on the pH of the solution. Nitrogen containing groups are particularly preferred because they further have a nucleophilic character.

Among these, polyallylamine hydrochloride (PAH) and poly(diallyldimethyl ammonium chloride) (PDADMAC), polyethylene imine (PEI), poly-L-histidine, poly-L-lysine and poly-4-vinylpyridine are particularly preferred.

L-poly-aminoacids are mentioned in particular because they are easily available, however D-polyamino-acids are equally appropriate.

The polycationic compound is used in solution, preferably in aqueous solution and particularly preferred in a buffered solution. The concentration of the solution is not critical, as long as the solution is stable. A concentration of about 0,1 to 1 mg/mL is generally suitable.

The term "mineral oxide precursors" is understood to cover compounds that are capable of reacting with the reactive groups of the polycationic compounds, for instance by a polycondensation reaction to yield, directly or indirectly, a mineral oxide. The term aims in particular at compounds that form mineral oxides in presence of cationic groups such as amine or imine groups.

Particularly preferred are mineral oxide precursors which are hydrosoluble. Hydrosoluble mineral oxide precursors may be used as aqueous solutions, and allow to avoid the use of solvents, which may be dangerous for human health when inhaled.

Particularly preferred hydrosoluble mineral oxide precursors are chosen from silicic acid and water soluble metal alkoxydes but metal complexes comprising anionic ligands such as titanium (IV) (bis ammoniumlactate) dihydroxyde may also be used.

There is no general limitation to the nature of the mineral oxide. However, at least in view of medical applications, the mineral oxide precursor is preferably a silicon compound or a titanium compound.

From the large groups of mineral oxide precursors, metal alkoxydes are particularly preferred.

Preferred alkoxydes are organometallic compounds having at least one group derived from an alcohol by removal of one H atom.

Alkoxydes tend to hydrolyse in presence of water to form metal hydroxides, which are generally unstable and polycondensate to yield oxides and water.

As set out above, one major difficulty implied with spray coating a mineral oxide precursor is the necessary limited presence of particles and thus the stability of the solution.

The term "stable solution" is intended to mean a solution which does not show a noticeable precipitation, as detected by the onset of turbidity for at least two hours under room temperature. The onset of turbidity was followed by UV-Vis spectroscopy at a wavelength of 350 nm for experiments performed with silicic acid.

Two different embodiments are proposed to stabilise the mineral oxide precursor solution. These embodiments may be used alone or in combination.

According to a first embodiment, the solution of the mineral oxide precursor is buffered to a pH such as to prevent precipitation, in particular spontaneous polycondensation.

The pH suitable for an optimal stability will depend on the nature of the mineral oxide precursor. By way of example, a solution of silicic acid or of titanium (IV) (bis ammoniumlactate) dihydroxyde can be stabilized at a pH of around 7,4. The use of 50 mM Tris buffer is thus appropriate for these precursors.

According to a second embodiment, the solution of the mineral oxide precursor has a concentration such as to prevent precipitation, in particular spontaneous polycondensation.

Generally, a low precursor concentration is favourable in terms of stability because it will slow down the precipitation. The determination of the optimal concentration is a matter of routine testing. As an example, a concentration of less than 30 mM has been found suitable for silicic acid while a concentration of up to 60 mM was found appropriate for bis(ammonium lactate) titanium dihydroxide.

The solutions may be provided in separate vessels equipped respectively with a spraying nozzle. It is also possible to provide the solutions through a spray coating device having a common spraying nozzle.

The substrate is preferably hold with an angle, preferably nearly or completely vertically during spray coating. Thereby, excess solution is eliminated easily by gravity.

After step ii), it can be advantageous to leave the substrate for some seconds in order to allow for the reaction between the mineral oxide precursor and the polycationic compound.

Although this is not mandatory, it is however preferred that the coated substrate is rinsed after step i) and step ii), respectively. The rinsing is normally done with the solvent of the solutions used. When working with aqueous solutions, the rinsing may be carried out conveniently using water.

Finally, while also not necessary, it can be advantageous to dry the substrate between the respective spray coating steps, for instance by air or dry nitrogen.

According to the concentration of the solution used, the layers have a varying thickness. Generally, the thickness of one layer will be lower than one micron. Preferably, the alternate layers A and B have a respective thickness of about 1 nm to about 100 nm. Particularly preferred is a thickness of about 10 to about 50 nm.

In particular in view of a very small thickness of each layer, it is necessary to repeat the steps i) and ii) a number of times before a continuous film is obtained. A continuous film is understood to mean to be formed upon percolation of the particles in the film. This can be determined easily by using a technique such as Atomic Force Microscopy.

The number of repetitions of steps i) and ii) necessary to obtain a continuous film will depend on the process parameters such as the concentration of the solution and the nature of the substrate. However, generally, the repetition of steps i) and ii) for between 10 and 100 times is appropriate to yield a continuous film.

The obtained homogeneous composite films are made from mineral oxide particles agglomerates and polycations. These films have a defined composition, thickness and structure. Generally, the total thickness of the films is from about 10 nm to about 1000 nm.

In contrast with pure polyelectrolyte films, the cohesion of the different layers in these films is not only due to electrostatic interactions, but also by bonds created by the chemical reaction between the polycationic compound and the mineral oxide precursor.

A second aspect of the invention concerns a substrate coated with a composite film of alternate layers A and layers B, wherein layer A comprises or is made of a polycationic compound and layer B comprises or is made of a mineral oxide.

Preferably, the first layer on the substrate is a layer A.

Indeed, most of the substrates are intrinsically ionic and adhesion is generally better when starting with an ionic layer.

In order to further improve adhesion, it may be contemplated to provide a primer film between the substrate and the composite film. Such a primer film may in particular comprise a polyanionic layer. Particularly preferred is a primer film consisting of a polyelectrolytic layer obtained by depositing alternately a polyanionic and a polycationic compound. An appropriate multilayer primer film typically consists of 2 to 20 layers.

The substrate may be a solid made of or comprising a metal oxide, a glass, or a polymer.

Particularly useful as substrates are biomaterials, that is materials that do not elicit a negative response when put in contact with biological tissues, and especially materials that are intended to be placed within the living body, in particular implants and prosthesis.

The described method can also be used on glass substrates to create anti-bacterial substrates. These films can also find applications in photovoltaic cells.

The substrate can be used as such or eventually beforehand coated with a primer film. Such a primer film can be for instance a polyelectrolytic film formed of alternate layers of polycationic compounds such as polyethylene imine (PEI) or polyaminoacid and polyanionic compounds such as poly-4-styrene sodium sulfonate (PSS). Such a primer film can be for instance a PEI (PSS-PAH)₅ architecture.

Advantageously, the primer film is deposited by spray coating.

The composite films thus obtained are intrinsically porous.

This particular feature allows for the inclusion of active species in the film, which may then be released progressively, in particular within the body. The composite films may in particular contain one or more pharmaceutically active species. Such active species may be for instance drugs or enzymes.

Composite films thus obtained display selective permeability towards small anions whereas pure mineral oxides such as silica gel is permeable towards small cations. This shows that the sign of the fixed charges in the composite material has been reversed from negative to positive upon polycation incorporation. Indeed, titanium dioxyde particles for instance are negatively charged at pH 7.4.

The composite film may, as described above, be coated on top of a primer film. Further, according to another embodiment, the composite film may further be coated with another film, in particular with a polyelectrolyte film.

It is thereby in particular possible to include a composite film as described between two polyelectrolyte films. This offers interesting perspectives to improve the mechanical properties of polyelectrolyte films.

The composite films prepared according to the disclosed method comprise mineral oxide particles of nanometrical size and may therefore also be useful as optical films.

For instance, composite films obtained with titanium oxide precursors can be useful as UV filters since the particles absorb light in the UV region, while having a high transmission in the visible region.

Also, the composite films may be used with suitable mineral oxides as high refractive index films.

The composite films may also be useful as photocatalytic films, in antibacterial films, and for the preparation of photovoltaic cells.

The composite films are porous and present an excess of immobile electrical charges compensated by small anions. Therefore, they may also be useful for filtration purposes, such as for water filtration.

The invention will be explained more in detail by the following examples and the accompanying figures, which show:
- Fig.1 :: the absorbance at 350 nm of silicic acid solutions with different concentrations and after adding PAH;
- Fig.2a:: the optical thickness of a film obtained according to the invention (o, ■, △) and using silicic acid spray deposition only (•);
- Fig.2b:: the thickness increment per layer pair of a composite film obtained by spray deposition of silicic acid and PAH as a function of the concentration of the silicic acid solution used;
- Fig. 3 :: the thickness of a composite film obtained according to example 6 using different mineral oxide precursors;
- Fig. 4 :: ATR-FTIR spectroscopy data of a PEI-(PSS-PAH)₅-(silicic acid-PAH)ₙ film obtained according to example 9, with n = 0, 1, 2 and 3 and with silica particles deposited by solvent casting from an ethanolic solution (shifted by 0,12 absorbance unit for the sake of clarity);
- Fig.5a :: AFM image acquired by scanning perpendicularly to a scratched line on a PEI-(PSS-PAH)₅-(silicic acid-PAH)₁₅ film according to example 1;
- Fig. 5b:: an ESEM micrograph of a PEI-(PSS-PAH)₂-(SiO₂-PAH)₆ film;
- Fig. 6:: a voltamogram obtained with a scan rate of 100 mV/s of a PEI-PSS-(PAH-silicic acid)₃₀ film according to example 10 in contact with (a) ferrocene methanol; (b) ferrocyanide, (c) ruthenium (III) hexamine;
- Fig. 7 :: the absorbance spectrum of a composite film according to example 11 on a quartz substrate for different numbers of layer pairs; and
- Fig. 8 :: Absorbance at 232 nm (○, left scale) and at 450 nm (■, right scale) as a function of the number of layer pairs and film layer thickness increase (◆, right scale).

### EXAMPLES

### Materials

All solutions were prepared form milli-Q ultrapure water (p= 18.2 MΩ.cm). All polyelectrolytes, silicic acid, and ferrocyanide solutions were freshly prepared before each deposition experiment. Ferrocyanide (K₄Fe(CN)₆·3(H₂O), Sigma ref. P9387, batch 103K0168) ruthenium(II)hexamine chloride (Aldrich, ref 303690, CAS number 15305-72), Ferrocene methanol (ref335061), poly(ethylene imine) (PEI, sigma, P3143, 093K0098) as 50% w/w solution, poly(styrene sulfonate) (PSS, cat. Number 24,305-1, Mw ≈ 70000 g/mol) and poly(allylamine hydrochloride) (PAH, Aldrich, Cat. Number 28, 322-3, Mw ≈ 70000 g/mol) were used without further purification. Silicic acid was purchased from Riedel De Haën (ref 13729, lot 1219A), in the form of water glass. All films were made with polyelectrolytes and chemical species dissolved in 0.05 mol.L⁻¹ Tris(hydroxymethyl amino methane), the pH of which was adjusted to 7.4. The pH of the silicic acid solution had to be adjusted again to 7.4 with concentrated hydrochloric acid after the dissolution of silicic acid whose mother solution contained 11 % NaOH. This high pH of the mother solutions prevents spontaneous polycondensation to produce some silica.

### Concentration range of the silicic acid solution

The concentration range of silicic acid that allows to spray monomers or small oligomers of silicic acid alternately with a polycation was defined by following the turbidity changes of silicic acid (at pH 7.4) as a function of time at a wavelength of 350 nm (at which neither silicic acid nor silica absorb light). An increase in turbidity means that colloidal silica particles are formed in solution. Such colloidal particles would then also form in the bottles in which silicic acid is stored before spraying. These control experiments were done in order to ensure that the deposited particles are not the result of an artefact.

Silicic acid solutions with a concentration between 0 and 0.04 M were prepared in a 0.05 M Tris buffer. After adjustment of the pH to 7.4, they were introduced into a 1 cm path quartz cuvette and the absorbance followed as a function of time at a wavelength of 350 nm with a time resolution of 1 min during at least three hours. This duration corresponded to the longest spray experiments.

Since neither silicic acid nor silica absorb light at 350 nm, any increase in absorbance corresponds to light lost by scattering and hence reflects the spontaneous appearance of silica particles.

At higher concentrations, the solutions appeared turbid just after pH adjustment. Hence the silicic acid solutions used for the spray deposition-reaction experiments had a lower concentration.

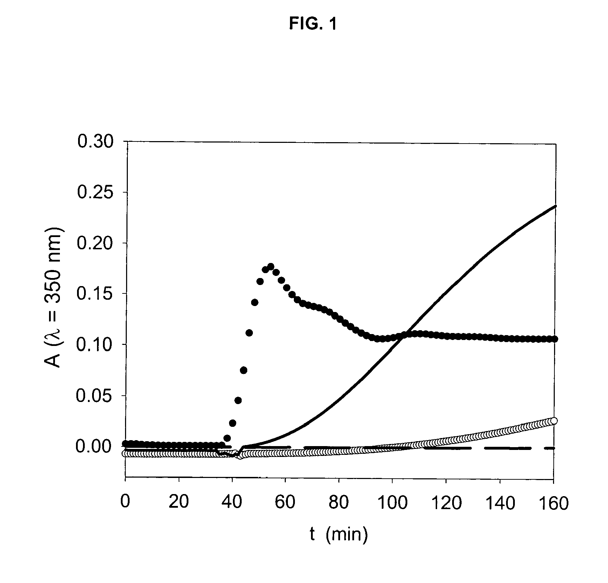

The turbidity evolution with time is represented for different concentrations of silicic acid in Fig. 1. It appears that colloidal particles are not formed for at least two hours as long as the silicic acid concentration is lower than 0.02 M. This duration is sufficient to deposit more than 40 pairs of silicic acid - PAH deposition. The fact that turbidity of the solution remains close to zero does not allow us to exclude that some small silica oligomers are growing in solution.

However, as soon as some PAH (to reach a final concentration of 0.04 mg/mL) is added under agitation to the 0.02 M containing silicic acid solution, a burst in turbidity is observed (followed by a slow decrease due to sedimentation of formed colloidal particles). This shows that the presence of a polycation induces a rapid polycondensation of silicic acid into silica particles and subsequently into a silica gel.

### Preparation of the substrate and characterisation by ellipsometry

Freshly cleaned (100) silicon wafers (Wafernet, INC, San Jose, CA) were used. The chips were cut rectangular, 4 cm x 1 cm in dimension, by means of a diamond knife.

The cleaning method consisted of an ethanol washing step, water rinse, immersion in a hot (~ 70°C) Hellmanex solution (2% w/w, Hellma, GmbH, Mülheim, Germany) during half an hour, followed by intensive MilliQ water rinse, immersion in a hot (~ 70°C) 1 M HCl solution during half an hour and a final MilliQ water rinse.

The thickness of the grown silicon oxide film atop the silicon substrate was measured by means of ellipsometry (Jobin Yvon, model PZ 2000, France) at a constant angle of incidence (70°) after nitrogen drying. At the working wavelength of 632.8 nm, the refractive index of silicon oxide was taken equal to 1.465. A single one layer Fresnel model was used to evaluate the data.

The silicon wafer was hydrated again and immersed in a 0.5 mg/mL positively charged PEI solution during 5 min to allow for the deposition of a primer polyelectrolyte later.

The substrate was then immobilised in a close to vertical orientation and hold with a tweezer on its upper part to allow for optimal liquid drainage upon spraying. The(PSS-PAH)₅ polyelectrolyte multilayer film was then deposited by spraying each polyelectrolyte solution (at 0.5 mg/mL in 0.05 M Tris buffer) during 5s and allowing for further polyelectrolyte solution drainage during an additional duration of 15s. The buffer solution was then sprayed also for 5s with a waiting time of 15s.

The thickness of the PEI-(PSS-PAH)₅ multilayer was then determined by means of ellipsometry after water rinse (to avoid the appearance of crystals from the buffer molecules) and drying under a nitrogen flow. For all the evaluations of the ellipsometry measurements, we assumed the deposited film to have a refractive index of 1.465, which in the case of PEI-(PSS-PAH)₅ is very close to the values obtained in situ by means of Brewster angle reflectometry and optical waveguide lightmode spectroscopy.

However, it should be kept in mind that ellipsometry allows for the accurate calculation of n*_{film}*.d were n*_{film}* and d are the refractive index and the geometrical thickness of the adlayer, hence any overestimation in n*_{film}* results in a underestimation of d. For this reason the film thickness was also determined by atomic force microscopy in the dry state after needle scratching, as will be described later.

### EXAMPLE 1

### Preparation of a silicic acid-PAH composite film and characterisation by ellipsometry

Onto a substrate prepared according to the procedure disclosed above, a composite silica-polycation film was deposited by alternately spraying a silicic acid solution at 10,8 mM and a polycation solution of polyallylamine hydrochloride (PAH) which was kept at 0.5 mg/mL. This process is also called reactive spray deposition.

Each species was sprayed during 5s with an additional 15s of drainage. Between each deposition, Tris buffer was sprayed during 5s to wash away any non reacted and deposited species. Buffer drainage was also allowed during 15 s.

In most experiments, the silicon chip with the deposited material was water rinsed, dried with a nitrogen flow, and characterized by ellipsometry every two deposition-reaction cycles, where a deposition-reaction cycle corresponds to the spraying of silicic acid and the polycation. Before continuing the reactive spray deposition, the film was rehydrated again with a short pulse (about 2s) of Tris buffer spray.

In order to ensure that these drying-rehydration cycles did not disturb the deposition of the investigated composite, some experiments were also performed by depositing material during 10 deposition reaction cycles without intermediate drying.

The total thickness of the obtained composite was then compared to the thickness of a PEI-(PSS-PAH)₅ film onto which two deposition-reaction cycles were performed five times with intermediate drying steps. Every value of the film thickness corresponds to the average over 5 measurements performed on regularly spaced points along the main axis of the rectangular silicon chip along which solution drainage occurred.

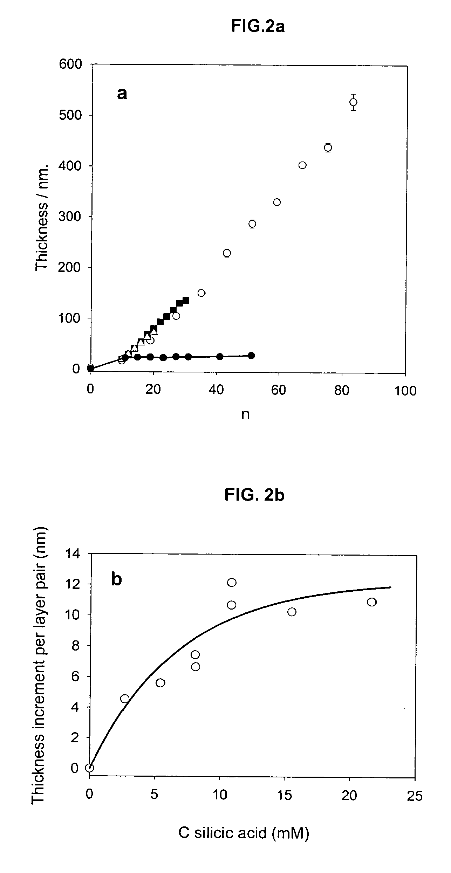

The optical thickness of the deposited material increases linearly with the number of spraying steps (either silicic acid or PAH). This is reproducible as long as the temperature of the room (here 25 ± 2°C) and the pressure inside the spraying cans are maintained constant (Fig. 2b).

### EXAMPLE 2

### Influence of the silicic acid concentration

In order to evaluate the effect of silicic acid concentration in the spraying cans on the slope of the optical thickness versus layer number curves, example 1 was repeated by varying the concentration of the silicic acid between 0 and 25 mM.

The evolution of the optical thickness was measured by ellipsometry, according to the procedure described above. The results are illustrated in Fig. 2b. It appears that the slopes increase to reach a plateau for silicic acid concentrations higher than about 10 mM (Fig. 2b (•)).

### EXAMPLE 3 (COMPARATIVE)

### Preparation of a silicic acid-PAH composite film by dipping and characterisation by ellipsometry

In order to evaluate the influence of the coating method, example 1 is repeated, except that instead of spray coating, the silicic acid and PAH are deposited alternatively by the classical dipping method.

The evolution of the optical thickness was measured by ellipsometry, according to the procedure described above. The results are illustrated on Fig. 2b. The thickness increment per layer is smaller than by the spray deposition method (Fig. 2b, □).

### EXAMPLE 4 (COMPARATIVE)

### Preparation of a composite film without polycationic compound

The example 1 is reproduced, except that the silicic acid is sprayed onto a silicon wafer in alternation with Tris buffer, without a polycation.

The optical thickness of the film remains constant and equal to its value at the end of the first silica deposition (Fig.2a (•)).

This can be explained by the hypothesis that that the excess of amino groups afforded by the polycation deposition induces the polycondensation from silicic acid provided by the spray. If some charge reversal occurs then (silica particles will be negatively charged at pH 7.4) the reactive layer by layer spray coating could be continued. Indeed electrostatic layer-by-layer coating is driven through charge inversion at each coating step.

### EXAMPLE 5

### Preparation of a composite film coated with a polyelectrolyte film

The process of example 1 is repeated, except that the composite film obtained was further coated with a polyelectrolyte PSS-PAH film.

A film having the following layer structure is obtained: PEI-(PSS-PAH)₁₂-(silicic acid-PAH)₁₂-(PAH-PSS)₁₂.

The evolution of the optical thickness was measured by ellipsometry, according to the procedure described above.

The thickness increment per layer of the top film (0.83 nm per layer) is very close to the thickness increment per layer in the PEI-(PSS-PAH)₅ precursor film (0.78 nm per layer) and significantly different from the optical thickness increment in the reactive silicic acid-PAH deposition (1.9 nm per layer).

These experiments show that it is possible to include strata of composite silica - polyelectrolyte by this reactive deposition between two regular polyelectrolyte films. This may offer some interesting perspectives to improve the mechanical properties of the polyelectrolytes multilayers.

### EXAMPLE 6

### Preparation of a silicic acid-PDADMAC composite film and characterisation by ellipsometry

The example 1 is repeated, except that the polycation solution of PAH is replaced by a polycation solution of polydiallyldimethyl ammonium chloride (PDADMAC) of the same concentration. Further, a silicic acid solution of a concentration of 0,5 mg/mL is used.

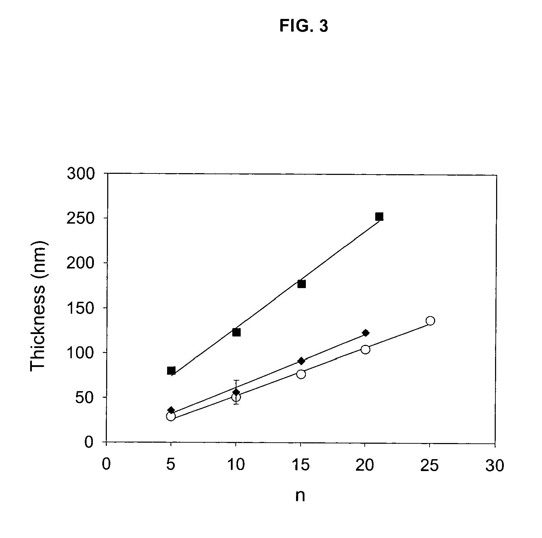

The evolution of the optical thickness was measured by ellipsometry, according to the procedure described above. The results are illustrated on Fig. 3. The thickness increment of the PDADMAC - silicic acid composite film is 5.6 nm/layer.

As a comparison, the thickness increment of a film obtained with a PAH solution in the same conditions is 6.0 nm/layer.

### EXAMPLE 7

### Preparation of a silicic acid-PEI composite film and characterisation by ellipsometry

The example 1 is repeated, except that the polycation solution of PAH is replaced by a polycation solution of polyethylene imine (PEI) of the same concentration. Further, a silicic acid solution of a concentration of 10.8 mM is used.

The evolution of the optical thickness was measured by ellipsometry, according to the procedure described above. The results are illustrated on Fig. 3. The thickness increment of the PEI - silicic acid composite film is 4.3 nm/layer.

### EXAMPLE 8

### Preparation of a silicic acid-poly-L-lysine composite film and characterisation by ellipsometry

The example 1 is repeated, except that the polycation solution of PAH is replaced by a polycation solution of poly-L-lysine at 0.5 mg/mL. Further, a silicic acid solution at 10.8 mM is used.

The evolution of the optical thickness was measured by ellipsometry, according to the procedure described above. The results are illustrated on Fig. 3. The thickness increment of the poly-L-lysine - silicic acid composite film is 2.3 nm/layer.

### EXAMPLE 9

### Characterisation of the composite film by ATR-FTIR

A composite film comprising PEI-(PSS-PAH)₅-(silicic acid-PAH)₁₀ was prepared according to Example 1, but replacing the silicium wafer with a ZnSe crystal for the infrared spectroscopy. It has to be noted that these experiments were performed by alternately circulating a polycation and a silicic acid solution a top the ZnSe crystal. But the same result can be obtained by the spraying deposition method.

### Infrared spectroscopy in the ATR mode

Fourrier transformed infrared spectra in the attenuated total reflection mode (ATR-FTIR spectra) were acquired on a trapezoidal ZnSe crystal before and after the reactive spray deposition by adding 512 interferogramms at 2 cm⁻¹ spectral resolution. To this aim we used a liquid nitrogen cooled mercury cadmium detector on a Bruker Equinox 55 spectrometer (Bruker, Wissembourg, France).

The spectrum before film deposition was taken as the reference spectrum and the absorbance was calculated as the logarithm of the ratio between the transmitted intensity after film deposition and the transmitted intensity before film deposition. The spectra of PEI-(PSS-PAH)₅ were subtracted from the spectra acquired after each step of the deposition process.

### Atomic force microscopy

In order to check the uniformity of the surface coverage due to the PEM film deposition, atomic force microscopy experiments were performed in the dry state for films deposited at a silicic acid concentration of 10.8 mM and PAH concentration of 0.5 mg/mL. These films were prepared as previously described on silicon wafers and were made from either 6 or 15 pairs of (silicic acid-PAH) depositions.

The surfaces were imaged in the contact mode with a Nanoscope IV microscope (Veeco) using silicon nitride cantilevers and tips with a nominal spring constant of 0.05 N m⁻¹. Just before imaging, the film was rapidly rinsed with distilled water, blown dry with a stream of nitrogen and scratched with an ethanol cleaned syringe needle. The film was then imaged dry (or wet in presence of a NaCl solution) in the contact mode in a scan direction perpendicular to the scratched line. Before image acquisition, the stability of the visualized structures was checked by allowing several scans over the investigated area.

### Environmental scanning electron microscopy (ESEM)

Multilayer films were sprayed as previously described onto cleaned glass slides, air dried and subsequently sputter-coated (Hummer-Junior, Siemens, Germany) with gold.

Secondary electron imaging was performed by using scanning electron microscopy FEI Quanta 400, FEI Company^{™}, Hillsboro, Or USA) operating in the high-vacuum mode with an acceleration voltage of the electrons of 30 kV.

The results of the characterisation show that the film contains silica.

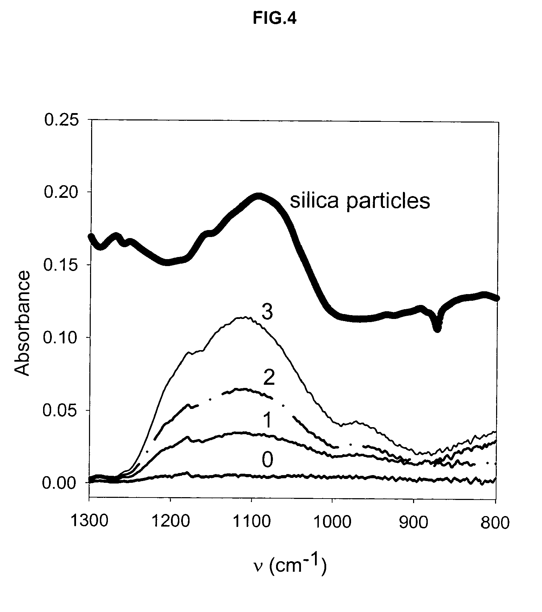

A closer inspection of Fig. 4 reveals that the shape of the difference spectra between the PEI-(PSS-PAH)₄-(silicic acid-PAH)ₙ deposits and those of the precursor film PEI(PSS-PAH)₄ which are taken as a new reference spectra, are very close to those of a thin layer of silica particles. However, the bands attributed to the silica stretching modes are shifted to higher wavenumbers, by about 30 cm⁻¹, with respect to those of pure silica particles (directly deposited on the surface of a cleaned ZnSe ATR crystal), whose main band is located at 1085 cm⁻¹.

This is in agreement with published data (T.Mizutani, H.Nagase, N.Fujiwara, H.Ogoshi, Bull. Chem. Soc. Jpn. 1998, 71, 2017). This points to the fact that the interaction with the PAH molecules slightly modifies the Si-O stretching vibrations.

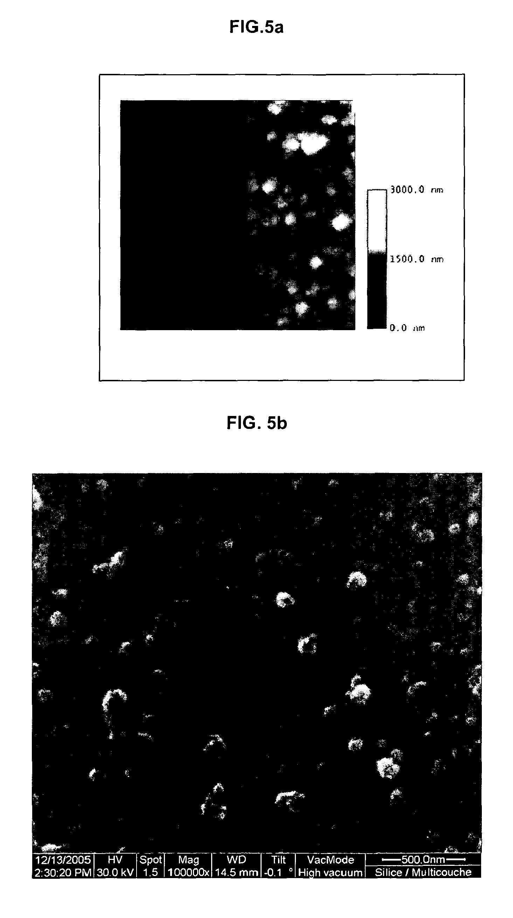

In addition, if deposition is performed in conditions corresponding to the beginning of the plateau in Fig. 2b (at about 10 mM in silicic acid), AFM imaging of a needle scratched PEI-(PSS-PAH)₅-(silicic acid-PAH)₁₅ deposit revealed that a continuous, but rough, film is formed without holes extending down to the substrate (Fig. 5a).

Closer examination of the non scratched part of the film reveals the presence of granular morphology with emerging particles ranging between about 50 and 300 nm in diameter.

This was confirmed also by the ESEM image of a PEI-(PSS-PAH)₅-(silicic acid -PAH)₆ deposit (Fig. 5b).

However, lower resolution ESEM micrographs (Fig. 5c) reveal that 6 pairs of reactive layer by layer deposition steps are not sufficient to uniformly coat the PEI-(PSS-PAH)₅ covered substrate.

At 10.8 mM in silicic acid, further AFM pictures showed that the minimal number of reactive deposition steps to obtain a continuous film lies between about 8 and 12 (data not shown). Hence, it appears that the layer by layer spray coating of silicic acid and polycations according to the invention produces a film that is indeed a conglomerate of polydispersed silica particles. It might well be that PAH plays the role of an electrostatic glue holding these particles together and is also potentially incorporated into these particles.

### EXAMPLE 10

### Selective permeability and excess charges

Cyclic voltammetry experiments were performed with the aim to investigate both if the films display a selective permeability and if they carry an internal excess of non compensated charged groups.

For these experiments, the precursor film was made from only one PEI and one PSS layer because it has been demonstrated that PSS-PAH multilayers build up from solutions of small ionic strength become rapidly impermeable when increasing the number of deposited pairs of layers.

A (PAH-silicic acid)₃₀ multilayer was deposited on top of this precursor film by means of alternately dipping the gold electrode in the PAH and silicic acid solutions.

This deposition performed by alternatively dipping the gold electrode in the PAH and silicic acid solutions, is sufficient to build up a continuous film and the oxidation and reduction currents that may be measured are then due to electroactive species that permeate through the film.

All electrochemical equipment used was purchased from CH instruments: it is made from a potentiostat model 604B with a Farraday cage linked to a regular 3 electrodes system. The Ag/AgCl/KCl (3 mol.L⁻¹) reference electrode and a platinum wire as counter electrode were coupled with gold working electrodes 2 mm in diameter. These gold working electrodes were cleaned before each experiment according to the following protocol: three polishing cycles with aluminum oxide powder solutions of 1, 0.3 and 0.05 µm granulometry, respectively. Each polishing cycle lasted over 2 min. It was followed by ultrapure water rinsing and repeated two additional times and finally sonicating the electrode twice. Finally the working electrodes were stored in water.

Just before the film deposition, the working electrodes were submitted to an electrochemical treatment, consisting in applying 1000 complete cycles between 0.2 and 1.6 volts at a scanning rate of 10 V.s⁻¹. This electrochemical treatment was performed in a 0.5 mol.L⁻¹ sulphuric acid solution.

Contrary to all other previously described characterisation methods, film deposition on treated working electrodes was performed by a conventional dipping layer-by-layer method, since it was impossible to control spray deposition on the 2 mm diameter gold electrodes.

The precursor layer was formed by successive dipping in PEI (1 mg/mL) solution, buffer rinsing, dipping into a 0.5 mg/mL PSS solution and a final buffer rinse. The further deposition of a (PAH-(silicic acid)ₙ-PAH film was then performed using a dipping Robot (Riegler & Kirstein GmbH, Berlin, Germany), as described in detail elsewhere (C.Porcel, Ph.Lavalle, V.Ball, G.Decher, B. Senger, J.-C. Voegel, P. Schaaf, Langmuir, 2006, 22, 4376-4383).

For these experiments, each dipping, either in the PAH or in the silicic acid solution, lasted over 5 min. Each adsorption-reaction step was followed by rinsing in three dedicated buffer bechers : 20 times 5 seconds in the first one, 5 times 20 seconds in the second one and twice one minute in the third one. For the longest sequences (corresponding to the deposition of 30 or 45 PAH-silicic acid layer pairs), the deposition process was split in two in order to refresh all solutions. This deposition method led to about 24 hours of delay between the end of the electrode cleaning-restructuration and the beginning of the voltammetry experiment.

Voltammetry experiments consisted in potential cycling between -0.1 and 0.65 volts vs reference electrode at a scanning rate of 0.1 V.s⁻¹. The first scan was performed in the buffer solution. After 5 minutes resting, a series of potential scans was done at 5 min intervals in presence of a 10⁻³ M ferrocyanide or ruthenium hexamine solution (in presence of 50 mM Tris buffer) until reaching a steady value of the oxidation or reduction current.

Finally the release kinetics of the redox probe was investigated in presence of pure Tris buffer also by performing regularly spaced potential scans in the same potential interval.

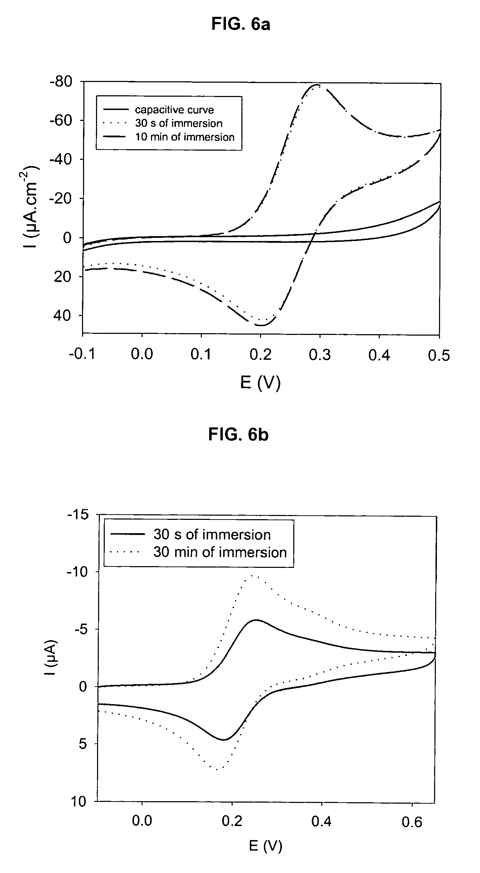

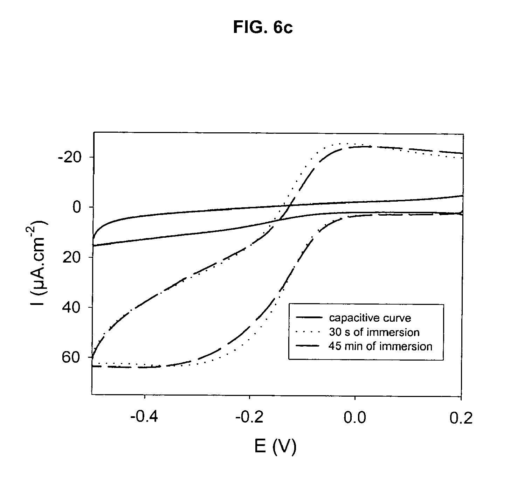

The film permeability was investigated with respect to three different electroactive compounds: the neutral ferrocene methanol (Fig. 6a), the anionic ferrocyanide (Fig. 6b) and the cationic ruthenium hexamine (Fig. 6c).

It appears clearly from the results that the composite film is highly permeable to ferrocene methanol. Indeed, the oxidation and reduction peaks of ferrocene methanol are symmetric and spaced by about 70 mV which is characteristic of a quasi reversible behaviour.

Further, the permeation in the films as well as the release kinetics are rapid (quantitative release from the film is achieved in less than ten minutes when the ferrocene methanol solution is replaced by pure Tris buffer).

When an identical film is put in contact with Tris buffer containing anionic ferrocyanide (1 mM Fe(CN)₆⁴⁻), the voltamograms are highly symmetric but the release kinetics in pure Tris buffer is slower than in the case of Ferrocene methanol.

Finally, when put in contact with ruthenium hexamine, the oxidation and reduction peaks are highly asymmetric and a steady state is reached very rapidly (in about 1 min.). In contrast, when the gold electrode covered with a PEI/PSS precursor layer is put in contact with a 1 mM ruthenium hexamine solution, the obtained oxidation and reduction peaks are typical of an equilibrium redox process. This means that either the mechanism of electron transfer between the gold electrode and this particular electroactive species is modified in presence of a PEI-PSS-(PAH-silicic acid)₃₀ film or that its permeation into the composite film is hindered.

Anyway, the permeation kinetics of the three investigated electroactive species into these films (about 300 nm thickness) is much more rapid than the permeation kinetics of ferrocyanide into much thicker polyelectrolyte films. This suggests that the density of ionic traps able to retain charged species is much more smaller in the composite films investigated here than in polyelectrolyte films

### EXAMPLE 11

### Preparation of a Ti(IV)(bisammonium lactate) dihvdroxvde-PDADMAC composite film

Onto a substrate prepared according to the procedure disclosed above, but without a primer film, a composite titanium dioxyde-polycation film was deposited by alternately spraying with Ti(IV)(bis ammonium lactate) dihydroxyde solution at 10 mM in a solution of 50 mM Tris buffer and a polycation solution of poly(diallyldimethylammonium chloride) (PDADMAC) which was kept at 0.5 mg/mL.

Each species was sprayed during 5s with an additional 15s of drainage. Between each deposition, Tris buffer was sprayed during 5s to wash away any non reacted and deposited species. Buffer drainage and reaction between the species was then allowed during 15 s.

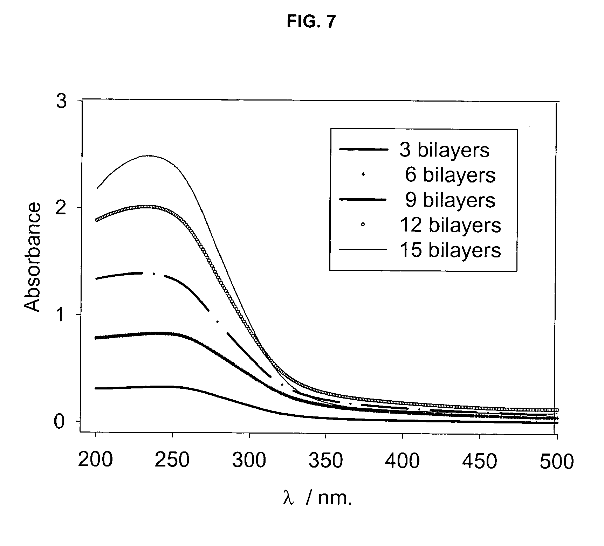

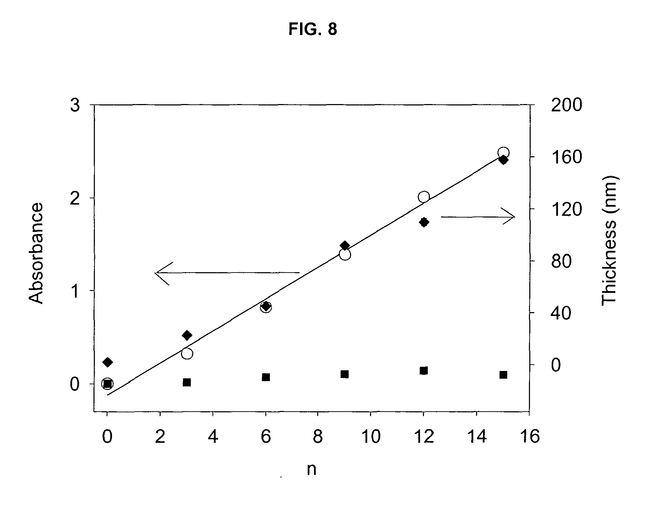

In order to follow the absorbance of the films, the same film was coated onto a transparent quartz substrate. The results are shown in Fig.7. II is noted that the film absorbance is very strong in the far UV region while it is weak in the visible region. Further, the absorbance at 232 nm as well as the optical thickness of the film increase linearly with the number of layer pairs (Fig. 8).

The thickness of the films obtained is remarkably homogeneous. Indeed, the thickness varies less than 10 nm over several cm² after coating 15 layer pairs, which leaves a film with a thickness of around 160 nm. This homogeneity is visible to the eye for the coated quartz glass.

As a comparison, a film was prepared by immersing a quartz glass alternately into a PDADMAC solution and a solution of TiO₂ (mean particle size 400 nm). The characterisation by UV-Vis spectroscopy and by ellipsometry shows that the obtained coating is less homogeneous than by spray coating. Further, the films are quite more opaque in the visible part of the spectrum (λ > 400 nm). It is concluded that the particles obtained by reactive spray coating are much smaller, of a nanometrical size and present thus an absorbance in the far UV rather than in the visible region.

It is thus possible to obtain mineral oxide-polycation composite films by alternatively spraying precursors of mineral oxide such as silica and different polycations. These films have been characterised with respect to their morphology and chemical composition. The obtained material displays high permeability to negatively charged ferrocyanide but the permeability to positively charged ruthenium hexamine is modified which suggests that the films contain an excess of positively charged polycation chains that do not directly interact with the spray produced mineral oxide particles.

This novel method of reactive layer by layer coating may be extended to different metal oxide precursors such as alkoxydes and should allow coating biomaterials with active bioceramics able to direct complex responses, like osteoconduction, *in vivo.*

## Claims

1. Method for providing a composite film onto a substrate comprising the steps consisting of:
i) coating onto a substrate a solution comprising a polycationic compound by spray coating, thus forming a layer A;
ii) coating onto the layer A an aqueous solution of a mineral oxide precursor by spray coating, thus forming a layer B; and
iii) repeating steps i) and ii) until the formed composite film has the desired thickness.

2. Method according to claim 1, wherein the substrate is an implant or a prosthesis.

3. Method according to claim 1 or 2, wherein the polycationic compound is chosen from polyamines polyimines and polyaminoacids.

4. Method according to claim 3, wherein the polycationic compound is PAH, PDADMAC, PEI, Poly-L-histidine; poly-L-lysine and Poly-4-vinylpyridine.

5. Method according to any of claims 1 to 4, wherein the mineral oxide precursor is hydrosoluble.

6. Method according to any of claims 1 to 5, wherein the mineral oxide precursor is a silicon compound or a titanium compound.

7. Method according to any of claims 1 to 6, wherein the mineral oxide precursor is a metal alkoxyde.

8. Method according to any of claims 1 to 7, wherein the mineral oxide precursor is chosen from silicic acid and titanium bis(ammonium)lactate dihydroxyde.

9. Method according to any of the claims 1 to 8, wherein the solution of the mineral oxide precursor is buffered to a pH such as to prevent precipitation.

10. Method according to any of the claims 1 to 9, wherein the solution of the mineral oxide precursor has a concentration such as to prevent precipitation.

11. Method according to any of the claims 1 to 10, wherein steps i) and ii) are repeated between 10 and 100 times.

12. Method according to any of the claims 1 to 11, wherein the coated substrate is rinsed after step i) and step ii), respectively.

13. Substrate coated with a composite film of alternate layers A and layers B, wherein layer A comprises a polycationic compound and layer B comprises a mineral oxide.

14. Substrate according to claim 13, wherein the first layer on the substrate is a layer A.

15. Substrate according to claim 13 or 14, wherein a primer film is provided between the substrate and the composite film.

16. Substrate according to claim 15, wherein the primer film comprises a polyanionic layer.

17. Substrate according to claim 16, wherein the primer film comprises a polyelectrolytic layer.

18. Substrate according to any of claims 13 to 17, wherein the alternate layers A and B have a respective thickness of about 1 nm to about 100 nm.

19. Substrate according to any of claims 13 to 18, wherein the composite film has a total thickness of from about 10 nm and about 1000 nm.

20. Substrate according to any of claims 13 to 19, wherein the composite film contains one or more pharmaceutically active species.

21. Substrate according to any of claims 13 to 20, wherein the composite film is further coated by a polyelectrolyte film.
